Europäisches Patentamt

European Patent Office

Office européen des brevets

(19)

(11) EP 0 743 923 B1

(12) FASCICULE DE BREVET EUROPEEN

(45) Date de publication et mention
de la délivrance du brevet:
**17.06.1998 Bulletin 1998/25**

(21) Numéro de dépôt: **95908988.9**

(22) Date de dépôt: **06.02.1995**

(51) Int Cl.[6]: **C01B 33/24**, C09C 1/28

(86) Numéro de dépôt international:
**PCT/FR95/00140**

(87) Numéro de publication internationale:
**WO 95/21791 (17.08.1995 Gazette 1995/35)**

(54) **SILICATES A BASE D'ALCALINE-TERREUX, DE CUIVRE ET EVENTUELLEMENT DE TITANE, PIGMENTS BLEUS OU VIOLETS A BASE DE CES SILICATES, LEUR PROCEDE DE PREPARATION ET LEUR UTILISATION**

AUF ERDALKALIELEMENTE, KUPFER UND GEGEBENENFALLS TITAN, BASIERTE SILICATE, BLAUE ODER PURPURFARBENE PIGMENTE DARAUF BASIEREND, VERFAHREN ZU DEREN HERSTELLUNG UND IHRE VERWENDUNG

SILICATES BASED ON ALKALINE-EARTH ELEMENTS, COPPER AND OPTIONALLY TITANIUM, BLUE OR PURPLE PIGMENTS BASED ON SAID SILICATES, PREPARATION METHOD THEREFOR AND USE THEREOF

(84) Etats contractants désignés:
**AT BE CH DE DK ES FR GB GR IT LI NL PT SE**

(30) Priorité: **11.02.1994 FR 9401560**

(43) Date de publication de la demande:
**27.11.1996 Bulletin 1996/48**

(73) Titulaire: **RHODIA CHIMIE**
**92408 Courbevoie Cédex (FR)**

(72) Inventeurs:
• **CHOPIN, Thierry**
**F-95320 Saint-Leu-la-Forêt (FR)**
• **MACAUDIERE, Pierre**
**F-92600 Asnières-sur-Seine (FR)**

(74) Mandataire: **Dubruc, Philippe et al**
**RHODIA CHIMIE**
**Direction de la Propriété Industrielle**
**25, quai Paul Doumer**
**92408 Courbevoie Cédex (FR)**

(56) Documents cités:
• **DATABASE WPI Week 8143 Derwent Publications Ltd., London, GB; AN 81-78569D & JP,A,56 115 360 (OKUMURA) , 10 Septembre 1981**
• **DATABASE WPI Week 9201 Derwent Publications Ltd., London, GB; AN 92-005395 & SU,A,1 623 951 (AS ARMN GEN INOR CH) , 30 Janvier 1991**
• **CHEMICAL ABSTRACTS, vol. 117, no. 25, 21 Décembre 1992 Columbus, Ohio, US; abstract no. 250667q, FITZHUGH ET AL 'a purple barium copper silicate pigment from early china' & STUD. CONSERV., vol. 37,no. 3, 1992 pages 145-154,**
• **CHEMICAL ABSTRACTS, vol. 86, no. 10, 7 Mars 1977 Columbus, Ohio, US; abstract no. 64732y, BAYER ET AL 'thermoanalytical study of the formation and decomposition of alkaline earth copper silicates' & TERMANAL '76,CELOSTATNA KONF. TERM. ANAL., 1976 pages 21-30,**
• **DATABASE WPI, semaine 8143, Derwent Publications Ltd., London (GB); AN 81-78569d & JP A 56115360 (OKUMURA), 10 September 1981**

**Description**

La présente invention concerne des silicates à base d'alcalino-terreux, de cuivre et éventuellement de titane, des pigments bleus ou violets à base de ces silicates, leur procédé de préparation et leur utilisation.

Les pigments minéraux de coloration sont déjà largement utilisés dans de nombreuses industries notamment dans celles des peintures, des matières plastiques et des céramiques. Dans de telles applications, les propriétés que sont, entre autres, la stabilité thermique et/ou chimique, la dispersabilité (aptitude du produit à se disperser correctement dans un milieu donné), la couleur intrinsèque, le pouvoir de coloration et le pouvoir opacifiant, constituent autant de critères particulièrement importants à prendre en considération dans le choix d'un pigment convenable.

Malheureusement, le problème est que la plupart des pigments minéraux qui conviennent pour des applications telles que ci-dessus et qui sont effectivement utilisés à ce jour à l'échelle industrielle, font généralement appel à des métaux (cadmium, plomb, chrome, cobalt notamment) dont l'emploi devient de plus en plus sévèrement réglementé, voire interdit, par les législations de nombreux pays, compte tenu en effet de leur toxicité réputée très élevée. On peut ainsi plus particulièrement citer, à titre d'exemples non limitatifs, le cas des pigments rouges à base de sélénure de cadmium et/ou de sulfosélénure de cadmium, et pour lesquels des substituts à base de sulfures de terres rares sont déjà maintenant proposés, ainsi que celui des pigments verts qui présentent quant à eux pour inconvénient de contenir le plus souvent du chrome sous la forme notamment de chromite de cobalt, de Victoria Green (grenats à base de chrome) ou d'oxyde de chrome III. On peut encore citer le cas des pigments bleus à base de cobalt.

On voit donc que la recherche, le développement et finalement la mise à disposition de nouveaux pigments minéraux de substitution constituent à ce jour un enjeu économique et industriel des plus importants.

On connaît par ailleurs des silicates de baryum et de cuivre notamment de couleur bleue ou violette mais la préparation de ces produits pose des difficultés. En effet, ces silicates sont obtenus généralement par chamottage. Dans ce cas, en raison des températures élevées exigées par ce type de procédé, on passe nécessairement par des phases fondues et on obtient de ce fait des produits essentiellement sous forme de verres. Ces verres présentent une coloration violette très foncée et sont d'un emploi très limité comme colorants. En effet, ils peuvent tout au plus être utilisés pour la coloration des céramiques ou des frittes car il est difficile de les broyer à une granulométrie inférieure à une dizaine de microns, granulométrie nécessaire pour la coloration d'autres matériaux, car un tel broyage entraîne généralement une altération trop importante de couleur qui les rend inutilisables.

Il existe donc un besoin certain en pigments colorés à base de silicates d'alcalino-terreux et de cuivre, de fine granulométrie, permettant la coloration d'une large gamme de matériaux et présentant aussi une gamme importante de couleurs notamment dans le bleu et le violet.

Un premier objet de l'invention est d'offrir un tel type de pigment.

Un second objet de l'invention est la mise au point d'un procédé de préparation de ce type de pigment.

Dans ce but, le silicate selon l'invention à base d'alcalino-terreux et de cuivre ou d'alcalino-terreux, de titane et de cuivre est caractérisé en ce qu'il se présente sous forme d'agglomérats constitués soit de grains monocristallins ou majoritairement monocristallins soit d'agrégats eux-mêmes constitués de particules monocristallines.

L'invention concerne aussi des silicates phasiquement purs et qui répondent à la formule $BaCuSi_2O_6$ ou la formule $BaCuSi_4O_{10}$.

L'invention couvre aussi les pigments colorés notamment de couleur bleue ou violette à base d'au moins un silicate du type précité.

Par ailleurs, le procédé de préparation d'un silicate selon l'invention est caractérisé en ce qu'il comporte les étapes suivantes :

- on mélange un sol de silice ou un silicate et éventuellement un sol de titane avec des sels des autres éléments constitutifs du silicate à l'exception de l'oxygène;
- on sèche le mélange ainsi obtenu;
- on calcine le produit obtenu.

D'autres caractéristiques, détails et avantages de l'invention apparaîtront plus clairement à la lecture de la description et des exemples concrets mais non limitatifs qui vont suivre.

Selon un premier mode de réalisation, la présente invention concerne des silicates à base d'un alcalino-terreux et de cuivre.

Selon un second mode de réalisation, les silicates de l'invention sont à base d'un alcalino-terreux, de titane et de cuivre.

Ici et pour le reste de la description on doit entendre par à base d'alcalino-terreux comme signifiant à base d'au moins un élément alcalino-terreux, un silicate de l'invention pouvant en effet comprendre dans sa formule plusieurs éléments alcalino-terreux.

Dans les deux cas, l'alcalino-terreux peut être plus particulièrement le baryum. Ce peut être aussi le calcium.

L'élément alcalino-terreux peut être partiellement substitué par une terre rare.

Par terre rare on entend ici les éléments du groupe constitué par l'yttrium et les éléments de la classification périodique de numéro atomique compris inclusivement entre 57 et 71. La classification périodique des éléments à laquelle il est fait référence est celle publiée dans le Supplément au Bulletin de la Société Chimique de France n° 1 (janvier 1966).

Selon un mode de réalisation préféré de l'invention, on utilise une terre rare légère, on entend par terre rare légère le lanthane, le cérium, le praséodyme et le néodyme. On peut citer tout particulièrement le lanthane.

Le cuivre peut aussi être partiellement substitué. L'élément de substitution peut être un alcalin, notamment le sodium ou le lithium. Ce peut être aussi le zinc, le nickel, le cobalt et le manganèse.

Il est possible dans le cadre de la présente invention d'avoir une substitution à la fois sur l'alcalino-terreux et le cuivre.

Les silicates de l'invention sont des silicates de cuivre ou des silicates de cuivre ou de titane. Ils peuvent répondre à la formule $MCuSi_2O_6$ ou $MCuSi_4O_{10}$ ou encore $MCuTiSi_3O_9$, M désignant l'alcalino terreux et M et Cu pouvant être substitués. Ces formules sont données à titre d'exemple et ne doivent pas être interprétées de manière limitative.

Les silicates de l'invention se caractérisent par leur constitution. Comme on l'a indiqué plus haut, ils se présentent sous forme d'agglomérats constitués soit de grains monocristallins ou majoritairement monocristallins soit d'agrégats eux-mêmes constitués de particules monocristallines.

Les agglomérats peuvent présenter une taille variable. Habituellement, leur taille moyenne est d'au plus 20µm, de préférence d'au plus 10µm. Cette taille est déterminée par granulométrie CILAS. Les grains monocristallins ou majoritairement monocristallins et les agrégats ont habituellement une taille moyenne qui varie entre 1 et 3µm. Les particules monocristallines qui constituent les agrégats ont elles une taille moyenne qui peut varier entre quelques angströms et quelques dizaines de nanomètres.

Les silicates de l'invention se présentent de préférence en outre sous forme d'une poudre.

Une propriété intéressante des silicates de l'invention réside dans le fait qu'ils peuvent être désagglomérés. On entend par là que la taille des agglomérats peut être très facilement réduite par un broyage dans des conditions douces c'est à dire en mettant en oeuvre par exemple un broyeur du type à jet d'air. Cette désagglomération qui permet d'atteindre les grains ou les agrégats constitutifs des agglomérats ne modifie pas couleur des produits d'une manière rédhibitoire.

Ainsi, les silicates de l'invention directement obtenus par le procédé qui sera décrit plus loin présentent après désagglomération une granulométrie voisine de la taille moyenne des grains ou des agrégats, c'est à dire d'au plus 5µm et plus particulièrement d'au plus 3µm.

Selon un mode de réalisation particulier de l'invention, les silicates de l'invention, outre leur finesse, ont aussi une granulométrie resserrée. Ainsi, leur indice de dispersion pour un produit désaggloméré ou non peut être d'au plus 1 et plus particulièrement d'au plus 0,7. Cet indice de dispersion est celui donné par le rapport $(\phi_{84}-\phi_{16})/2\phi_{50}$ dans lequel $\phi_{84}$, $\phi_{16}$, $\phi_{50}$ représentent les diamètres des particules correspondant à 84%, 16% et 50% de celles-ci.

Les silicates de l'invention présentent par ailleurs une large gamme de couleurs. Plus précisément, ils peuvent présenter les coordonnées chromatiques suivantes :

L* compris entre 25 et 80; a* compris entre -15 et +35; b* compris entre -5 et -55.

Les coordonnées chromatiques L*, a* et b* sont données ici et pour le reste de la description dans le système CIE 1976 (L*, a*, b*) tel que défini par la Commission Internationale d'Eclairage et répertorié dans le Recueil des Normes Françaises (AFNOR), couleur colorimétrique n° X08-12 (1983). Elles sont déterminées au moyen d'un colorimètre commercialisé par la Société Pacific Scientific. La nature de l'illuminant est D65. La surface d'observation est une pastille circulaire de 12,5 $cm^2$ de surface. Les conditions d'observations correspondent à une vision sous un angle d'ouverture de 10°. Dans les mesures données, la composante spéculaire est exclue.

$L^*$ donne une mesure de la réflectance (nuance clair/sombre) et varie ainsi de 100 (blanc) à 0 (noir).

a* et b* sont les valeurs des tendances colorées :

a* positif = rouge
a* négatif = vert
b* positif = jaune
b* négatif = bleu

L* représente donc la variation du noir au blanc, a* la variation du vert au rouge et b* la variation du jaune au bleu.

Des silicates plus particuliers selon l'invention vont maintenant être décrits.

Un de ces silicates répond à la formule (1) $BaCuSi_2O_6$ et il présente les coordonnées chromatiques suivantes :

L* >30; a* >15; b* <-30.

L'invention permet d'obtenir plus particulièrement un silicate de formule (1) non désaggloméré présentant les coordonnés chromatiques suivantes :

$$L^* =44; a^* =29; b^* =-50.$$

Un autre de ces silicates répond à la formule (2) $BaCuSi_4O_{10}$ et il présente les coordonnées chromatiques suivantes :

$$L^* >40; a^* <0; b^* <-30.$$

L'invention permet d'obtenir plus particulièrement un silicate de formule (2) non désaggloméré présentant les coordonnés chromatiques suivantes :

$$L^* =57; a^* =-2,5; b^* =-33.$$

En outre, les produits de formule (1) et (2) sont purs phasiquement. On entend ici par pur phasiquement des produits dont les spectres RX ne permettent de déceler que l'existence d'une phase unique dans des conditions d'analyse de routine.

Le procédé de préparation des produits de l'invention va maintenant être décrit.

Ce procédé comporte une première étape dans laquelle on mélange un sol de silice ou un silicate avec des sels des autres éléments constitutifs du silicate à l'exception de l'oxygène. Le cas échéant, pour la préparation d'un silicate comprenant du titane, on utilise aussi un sol de titane.

Le terme sol est utilisé ici dans le sens le plus commun comme désignant tout système constitué de fines particules solides de dimensions colloïdales en suspension dans une phase liquide généralement aqueuse.

Comme sol de silice, on peut utiliser ceux constitués de particules élémentaires de 100 à 500A°.

Comme sol de titane, on peut utiliser des sols présentant par exemple un pH compris entre 0,8 et 2,5 et constitués de cristallites élémentaires de $TiO_2$ de taille comprise entre 10 et 100A° agglomérés en amas submicroniques ayant des tailles de 200 à 1000A°.

Comme silicate, on peut mentionner les silicates organiques comme le silicate d'éthyle et les silicates d'ammonium quaternaire comme le silicate de tétraméthylammonium, de tétraéthylammonium, de tétrapropylammonium ou de tétrahydroxyéthylammonium.

On utilise par ailleurs des sels des éléments constitutifs du silicate autres que l'oxygène. On peut utiliser tout type de sel dans la mesure où celui-ci est soluble dans le mélange réactionnel et notamment dans la phase liquide du sol.

On utilise généralement des sels d'acides inorganiques comme les nitrates, chlorures ou sulfates. On préfère utiliser les nitrates.

Il est éventuellement possible d'utiliser les sels d'acides organiques, dans ce cas ceux-ci sont choisis plus particulièrement parmi les sels d'acides carboxyliques aliphatiques saturés ou parmi les sels d'acides hydroxycarboxyliques. On peut citer à titre d'exemple les formiates, les acétates, les propionates et les citrates.

Le mélange du ou des sols et des sels peut se faire dans un ordre quelconque par exemple par introduction du sol dans la solution contenant les sels ou inversement, l'ordre d'introduction pouvant dépendre de la stabilité du sol en fonction du pH. On travaille habituellement à température ambiante mais il est possible de chauffer le mélange.

Le mélange ainsi obtenu est ensuite séché.

Ce sechage peut se faire par tout moyen connu par exemple en étuve.

Toutefois, selon un mode de réalisation préféré de l'invention, ce séchage se fait par atomisation. c'est à dire par pulvérisation du mélange dans une atmosphère chaude (spray-drying). Ce type de séchage permet d'obtenir des produits à granulométrie fine et resserrée. L'atomisation peut être réalisée au moyen de tout pulvérisateur connu en soi, par exemple par une buse de pulvérisation du type pomme d'arrosoir ou autre. On peut également utiliser des atomiseurs dits à turbine. Sur les diverses techniques de pulvérisation susceptibles d'être mises en oeuvre dans le présent procédé, on pourra se référer notamment à l'ouvrage de base de MASTERS intitulé "SPRAY-DRYING" (deuxième édition, 1976, Editions Gerge Godwin - London).

A titre d'exemple, la température en début de séchage des gaz est habituellement comprise entre 200 et 300°C, celle de sortie peut varier entre 110 et 200°C. La pression peut être comprise par exemple entre 2 et 3 bars.

On notera que l'on peut également mettre en oeuvre l'opération d'atomisation-séchage au moyen d'un réacteur "flash", par exemple du type mis au point par la Demanderesse et décrit notamment dans les demandes de brevet français n° 2 257 326, 2 419 754 et 2 431 321. Dans ce cas, les gaz traitants (gaz chauds) sont animés d'un mouvement hélicoïdal et s'écoulent dans un puits-tourbillon. Le mélange à sécher est injecté suivant une trajectoire confondue avec l'axe de symétrie des trajectoires hélicoïdales desdits gaz, ce qui permet de transférer parfaitement la quantité de mouvement des gaz au mélange à traiter. Les gaz assurent ainsi en fait une double fonction : d'une part la pulvérisation, c'est à dire la transformation en fines gouttelettes, du mélange initial, et d'autre part le séchage des gouttelettes obtenues. Par ailleurs, le temps de séjour extrêmement faible (généralement inférieur à 1/10 de seconde environ) des particules dans le réacteur présente pour avantage, entre autres, de limiter d'éventuels risques de surchauffe par suite d'un contact trop long avec les gaz chauds.

Selon les débits respectifs des gaz et du mélange à sécher, la température d'entrée des gaz est comprise entre 400 et 900°C et plus particulièrement entre 600 et 800°C, la température du solide séché entre 150 et 300°C.

En ce qui concerne le réacteur flash mentionné plus haut, on pourra notamment se référer à la figure 1 de la demande de brevet français 2 431 321.

Celui-ci est constitué d'une chambre de combustion et d'une chambre de contact composée d'un bicône ou d'un cône tronqué dont la partie supérieure diverge. La chambre de combustion débouche dans la chambre de contact par un passage réduit.

La partie supérieure de la chambre de combustion est munie d'une ouverture permettant l'introduction de la phase combustible.

D'autre part la chambre de combustion comprend un cylindre interne coaxial, définissant ainsi à l'intérieur de celle-ci une zone centrale et une zone périphérique annulaire, présentant des perforations se situant pour la plupart vers la partie supérieure de l'appareil. La chambre comprend au minimum six perforations distribuées sur au moins un cercle, mais de préférence sur plusieurs cercles espacés axialement. La surface totale des perforations localisées dans la partie inférieure de la chambre peut être très faible, de l'ordre de 1/10 à 1/100 de la surface totale des perforations dudit cylindre interne coaxial.

Les perforations sont habituellement circulaires et présentent une épaisseur très faible. De préférence, le rapport du diamètre de celles-ci à l'épaisseur de la paroi est d'au moins 5, l'épaisseur minimale de la paroi étant seulement limitée par les impératifs mécaniques.

Enfin, un tuyau coudé débouche dans le passage réduit, dont l'extrémité s'ouvre dans l'axe de la zone centrale.

La phase gazeuse animée d'un mouvement hélicoïdal (par la suite appelée phase hélicoïdale) est composée d'un gaz, généralement de l'air, introduit dans un orifice pratiqué dans la zone annulaire, de préférence cet orifice est situé dans la partie inférieure de ladite zone.

Afin d'obtenir une phase hélicoïdale au niveau du passage réduit, la phase gazeuse est de préférence introduite à basse pression dans l'orifice précité, c'est-à-dire à une pression inférieure à 1 bar et plus particulièrement à une pression comprise entre 0,2 et 0,5 bar au-dessus de la pression existant dans la chambre de contact. La vitesse de cette phase hélicoïdale est généralement comprise entre 10 et 100 m/s et de préférence entre 30 et 60 m/s.

Par ailleurs, une phase combustible qui peut être notamment du méthane, est injectée axialement par l'ouverture précitée dans la zone centrale à une vitesse d'environ 100 à 150 m/s.

La phase combustible est enflammée par tout moyen connu, dans la région où le combustible et la phase hélicoïdale sont en contact.

Par la suite, le passage imposé des gaz dans le passage réduit se fait suivant un ensemble de trajectoires confondues avec des familles de génératrices d'un hyperboloïde. Ces génératrices reposent sur une famille de cercles, d'anneaux de petite taille localisés près et au-dessous du passage réduit, avant de diverger dans toutes les directions.

On introduit ensuite le mélange à traiter sous forme de liquide par le tuyau précité. Le liquide est alors fractionné en une multitude de gouttes, chacune d'elle étant transportée par un volume de gaz et soumise à un mouvement créant un effet centrifuge. Habituellement, le débit du liquide est compris entre 0,03 et 10 m/s..

Le rapport entre la quantité de mouvement propre de la phase hélicoïdale et celle du mélange liquide doit être élevé. En particulier il est d'au moins 100 et de préférence compris entre 1000 et 10000. Les quantités de mouvement au niveau du passage réduit sont calculées en fonction des débits d'entrée du gaz et du mélange à traiter, ainsi que de la section dudit passage. Une augmentation des débits entraîne un grossissement de la taille des gouttes.

Dans ces conditions, le mouvement propre des gaz est imposé dans sa direction et son intensité aux gouttes du mélange à traiter, séparées les unes des autres dans la zone de convergence des deux courants. La vitesse du mélange liquide est de plus réduite au minimum nécessaire pour obtenir un flot continu.

A l'issue de l'étape de séchage le produit obtenu qui est un précurseur du silicate de l'invention est calciné.

La température de calcination varie entre la température nécessaire pour former la phase silicate et celle au delà de laquelle on forme un verre. Cette température varie donc en fonction du type de silicate préparé. Habituellement elle est comprise entre 900 et 1100°C. Cette calcination se fait généralement sous air, toutefois, une calcination sous gaz inerte n'est pas exclue.

A l'issue de la calcination, on obtient des produits présentant une large gamme de couleurs pouvant varier du bleu au violet.

En outre, notamment dans le cas du séchage par atomisation, la granulométrie du produit récupéré est fine et régulière et est généralement d'au plus 20 microns de préférence d'au plus 10 microns. Il s'agit de la granulométrie des agglomérats.

Toutefois, il est tout à fait possible de diminuer cette granulométrie par simple désagglomération. On obtient ainsi des produits ayant, comme indiqué plus haut, une granulométrie d'au plus 5μm et plus particulièrement d'au plus 3μm.

Les silicates de l'invention peuvent être utilisés en tant que tels comme pigments ou entrer dans la composition de pigments colorés notamment bleus ou violets.

Les silicates ou pigments selon l'invention possèdent un très bon pouvoir de coloration et un très bon pouvoir opacifiant, et, de ce fait, conviennent à la coloration de nombreux matériaux, tels que plastiques, peintures et céramiques. A cet égard, la polyvalence des silicates ou pigments selon l'invention constitue l'un de leurs grands atouts.

Ainsi, et plus précisément encore, ils peuvent être utilisés dans la coloration de matières plastiques qui peuvent être du type thermoplastiques ou thermodurcissables.

Comme résines thermoplastiques susceptibles d'être colorées selon l'invention, on peut citer, à titre purement illustratif, le chlorure de polyvinyle, l'alcool polyvinylique, le polystyrène, les copolymères styrène-butadiène, styrène-acrylonitrile, acrylonitrile-butadiène-styrène (A.B.S.), les polymères acryliques notamment le polyméthacrylate de méthyle, les polyoléfines telles que le polyéthylène, le polypropylène, le polybutène, le polyméthylpentène, les dérivés cellulosiques tels que par exemple l'acétate de cellulose, l'acéto-butyrate de cellulose, l'éthylcéllulose, les polyamides dont le polyamide 6-6.

Concernant les résines thermodurcissables pour lesquelles les silicates ou pigments selon l'invention conviennent également, on peut citer, par exemple, les phénoplastes, les aminoplastes notamment les copolymères urée-formol, mélamine-formol, les résines époxy et les polyesters thermodurcissables.

On peut également mettre en oeuvre les silicates ou pigments de l'invention dans des polymères spéciaux tels que des polymères fluorés en particulier le polytétrafluoréthylène (P.T.F.E.), les polycarbonates, les élastomères silicones, les polyimides.

Dans cette application spécifique pour la coloration des plastiques, on peut mettre en oeuvre les silicates ou pigments de l'invention directement sous forme de poudres. On peut également, de préférence, les mettre en oeuvre sous une forme pré-dispersée, par exemple en prémélange avec une partie de la résine, sous forme d'un concentré pâte ou d'un liquide ce qui permet de les introduire à n'importe quel stade de la fabrication de la résine. Ce dernier point constitue un avantage particulièrement important des silicates ou pigments selon l'invention.

Ainsi, les silicates ou pigments selon l'invention peuvent être incorporés dans des matières plastiques telles que celles mentionnées ci-avant dans une proportion pondérale allant généralement soit de 0,01 à 5% (ramenée au produit final) soit de 40 à 70% dans le cas d'un concentré.

Les silicates ou pigments de l'invention peuvent être également utilisés dans le domaine des peintures et lasures et plus particulièrement dans les résines suivantes : résines alkydes dont la plus courante est dénommée glycérophtalique; les résines modifiées à l'huile longue ou courte; les résines acryliques dérivées des esters de l'acide acrylique (méthylique ou éthylique) et méthacrylique éventuellement copolymérisés avec l'acrylate d'éthyle, d'éthyl-2 hexyle ou de butyle; les résines vinyliques comme par exemple l'acétate de polyvinyle, le chlorure de polyvinyle, le butyralpolyvinylique, le formalpolyvinylique, et les copolymères chlorure de vinyle et acétate de vinyle ou chlorure de vinylidène; les résines aminoplastes ou phénoliques le plus souvent modifiées; les résines polyesters; les résines polyuréthanes; les résines époxy; les résines silicones.

Généralement, les silicates ou pigments sont mis en oeuvre à raison de 5 à 30% en poids de la peinture, et de 0,1 à 5% en poids du lasure.

Les silicates ou pigments de l'invention conviennent également pour la coloration des matières céramiques, comme par exemple les porcelaines, les faïences et les grès, et ceci soit par coloration à coeur de la céramique (mélange physique entre la poudre céramique et le pigment), soit par coloration uniquement de la surface de cette dernière au moyen de glaçures (compositions verrières de revêtement) contenant le pigment.

Dans cette application, la quantité de silicates ou pigments mise en oeuvre est généralement comprise entre 1 et 30% en poids par rapport soit à l'ensemble de la céramique, soit par rapport à la glaçure seule.

Enfin, les silicates ou pigments selon l'invention sont également susceptibles de convenir pour des applications dans l'industrie du caoutchouc, notamment dans les revêtements pour sols, dans l'industrie du papier et des encres d'imprimerie, dans le domaine de la cosmétique, ainsi que nombreuses autres utilisations comme par exemple, et non limitativement, la teinture, le finissage des cuirs et les revêtements stratifiés pour cuisines et autres plans de travail.

En ce qui concerne plus particulièrement la cosmétique, les produits de l'invention peuvent être utilisés dans la préparation de compositions pour maquillage et notamment la préparation de fards à yeux et de fards à joues. Ces fards peuvent se présenter sous forme de fards secs ou de fards gras. La teneur en pigments dans de tels fards peut varier dans de larges limites par exemple entre 2 à 20% en poids. Les fards secs sont des poudres à base par exemple de talc, de carbonate de magnésium, de stéarate de zinc, d'oxyde de zinc, de kaolin, de silicate de magnésium et d'aluminium qui sont chargées en pigment et agglomérées soit avec de la méthyl cellulose soit avec des stéarates. Les produits de l'invention peuvent rentrer aussi dans la composition de crayons de maquillage.

La présente invention couvre enfin les compositions de matière colorées notamment du type plastiques, peintures, lasures, caoutchoucs, céramiques, glaçures, papiers, encres, produits cosmétiques, teintures et revêtements stratifiés, qui comprennent des silicates ou des pigments colorés selon l'invention.

Des exemples vont maintenant être donnés.

EXEMPLE 1

Cet exemple illustre la préparation de $BaCuSi_4O_{10}$.

Dans 400 ml d'eau épurée, on mélange 32,66 g de nitrate de baryum $[(Ba(NO_3)_2;$ M = 261,35] et 30,2 g de nitrate de cuivre $[Cu (NO_3)_2, 3H_2O,$ M = 241,60].

On chauffe vers 60°C. On rajoute 75 g de sol de silice (Ludox à 40% en $SiO_2$).

On atomise le mélange dans un atomiseur BUCHI.

Conditions d'atomisation :
- débit 600 ml/h
- température entrée des gaz : 245°C
- température sortie des gaz : 128°C

On calcine ensuite le produit séché.

Conditions de calcination : 3 heures à 1050°C (300°C/h)

La poudre obtenue de couleur bleue présente les caractéristiques suivantes :

- diagramme RX: $BaCuSi_4O_{10}$
- Couleur :

  L* = 57
  a* = - 2,5
  b* = - 33

- diamètre $\phi$50 = 7,9 $\mu$m, ramené à moins de 2$\mu$m par simple désagglomération au jet d'air.

EXEMPLE 2

Cet exemple illustre la préparation d'un silicate du type de l'exemple 1 mais dopé au lanthane et au sodium et de formule $Ba_{0,5}La_{0,5}Cu_{0,5}Na_{0,5}Si_4O_{10}$

Dans 400 ml d'eau épurée, on mélange 32,66 g de nitrate de baryum, 43,7 ml d'une solution 2,86 M de nitrate de lanthane (d = 1,7, soit 74,3 g), 30,2 g de nitrate de cuivre et 10,6 g de nitrate de sodium.

On chauffe vers 60°C en agitant, puis on ajoute 60 g de $SiO_2$ (150 g de sol de Ludox à 40% de $SiO_2$).

On atomise le mélange dans un atomiseur BUCHI.

Conditions d'atomisation :
- débit 700 ml/h
- température d'entrée : 240°C
- température de sortie : 115°C

On calcine ensuite le produit.

Conditions de calcination : 2 heures à 950°C.

La poudre obtenue de couleur bleue présente les caractéristiques suivantes :

- diagramme RX : mélange de phases

  - $La_2Si_2O_7$
  - $BaCuSi_4O_{10}$
  - $SiO_2$ cristobalite (trace)

- couleur :

  L* = 58
  a* = 5
  b* = - 43

7

- diamètre $\phi 50 = 10$ µm ramené à 2-3µm par simple désagglomération au jet d'air.

EXEMPLE 3

Cet exemple illustre la préparation du $BaCuSi_2O_6$.
Dans 300 ml d'eau épurée, on mélange 32,66 g de nitrate de baryum [Ba $(NO_3)_2$, $3H_2O$, M = 241,60].
On chauffe vers 60°C. On rajoute 37,5 g de sol de $SiO_2$ (Ludox à 40% en $SiO_2$).
On atomise le mélange dans un atomiseur BUCHI.

Conditions d'atomisation :
- débit solution : 800 ml/h
- température d'entrée : 240°C
- température de sortie : 120°C

On calcine le produit obtenu.
Conditions de calcination : 2 heures à 950°C (300°C/h)
La poudre obtenue de couleur violette présente les caractéristiques suivantes :

- diagramme RX : $BaCuSi_2O_6$ quadratique
- couleur:

    L=44
    a = 29
    b = - 50

- diamètre $\phi 50$ : 5,8 µm, $\sigma/m = 0,9$ (indice de dispersion)
- après broyage jet d'air :
- couleur :

    L* = 48
    a* = 25
    b* = - 45

- diamètre $\phi 50$ : 1,75 µm, $\sigma/m = 0,7$ (indice de dispersion).

EXEMPLE 4

On prépare un silicate de formule $Ba_{0,75}Ca_{0,25}CuSi_2O_6$ en procédant selon le mode opératoire de l'exemple 3 et en introduisant les réactifs en quantité stoechiométrique. Le calcium est apporté sous forme de nitrate.
Le produit obtenu non broyé a un diamètre moyen inférieur à 3µm et présente une couleur bleue violette. Ses coordonnées chromatiques sont les suivantes :

    L* = 57,9
    a* = 5,4
    b* = -32,6

EXEMPLE 5

On prépare un silicate de formule $BaCu_{0,5}Zn_{0,5}Si_2O_6$ en procédant selon le mode opératoire de l'exemple 3 et en introduisant les réactifs en quantité stoechiométrique. Le zinc est apporté sous forme de nitrate.
Le produit obtenu non broyé a un diamètre moyen de 2,4µm et présente une couleur bleue violette. Ses coordonnées chromatiques sont les suivantes :

    L* = 66,3
    a* = 12,1
    b* = -32,5

EXEMPLE 6

Cet exemple illustre la préparation du silicate de formule $Ba_{0,9}Cu_{0,1}TiSi_3O_9$.

29,4 g de nitrate de baryum, 3,02 g de nitrate de cuivre et 49,3 g de sol de titane à 20,26% de $TiO_2$ sont dissous dans 350 ml d'eau vers 60°C.

On rajoute 56,25 g de sol de silice à 40% en $SiO_2$ et on atomise le mélange dans un atomiseur BUCHI.

Conditions d'atomisation :
- température d'entrée : 239°C
- température de sortie : 122°C
- débit de solution : 800 ml/h.

La poudre obtenue est calcinée 2 heures à 1000°C.
On obtient une poudre de couleur violette.

- Couleur :

  L* = 76
  a* = 12
  b* = - 20

- diamètre $\phi 50$ = 4,7 $\mu$m, $\sigma$/m = 0,56. On peut facilement ramener le diamètre moyen à moins de 2 $\mu$m par simple désagglomération au jet d'air.

EXEMPLE 7

Cet exemple illustre la préparation d'un silicate de formule $Ba_{0,5}Nd_{0,5}Cu_{0,5}Na_{0,5}Si_2O_6$

Dans un bécher de 2l, on ajoute 32,66g de nitrate de baryum soit 0,125 mole de Ba; 84,6g d'une solution de nitrate de néodyme à 21,3% en Nd soit 0,125 mole de Nd; 30,2g de nitrate de cuivre soit 0,125 mole de Cu; 10,62g de nitrate de sodium soit 0,125 mole de Na; 75g d'un sol à 40% de silice soit 0,5 mole de $SiO_2$ et 800ml d'eau déminéralisée.

On chauffe à 60°C et on atomise en maintenant le mélange à cette température.

La poudre obtenue est calcinée 3 heures à 850°C. Le produit non broyé a un diamètre moyen de 10$\mu$m et présente les coordonnées chromatiques suivantes :

  L* = 41,8
  a* = 12,1
  b* = -32,3

EXEMPLE 8

Cet exemple illustre la préparation d'un silicate de formule $Ba_{0,5}La_{0,5}Cu_{0,5}Na_{0,5}Si_2O_6$. On procède selon le mode opératoire de l'exemple 7 et en introduisant les réactifs en quantité stoechiométrique. Le lanthane est apporté sous forme de nitrate.

Le produit obtenu après calcination 2 heures à 950°C présente un diamètre moyen de 5$\mu$m et les coordonnées chromatiques suivantes :

  L* = 58
  a* = 5,1
  b* = -43

EXEMPLE 9

Cet exemple a pour but d'illustrer l'aptitude des pigments selon l'invention pour la coloration des plastiques.

20 g d'un pigment tel que préparé à l'exemple 3 sont mélangés en cube tournant à 2 Kg d'un polypropylène de référence Eltex® P HV 001.

Le mélange est ensuite extrudé à 180°C au moyen d'une extrudeuse bi vis ZSK 30 (commercialisée par la Société Wermer et Pfeiderer).

Les granulés obtenus sont ensuite injectés à 220°C au moyen d'une presse à injecter Arburg 350-90-220 D avec un cycle de 41 secondes.

Le moule est maintenu à la température de 35°C.

On obtient ainsi une éprouvette parallépipèdique à double épaisseur (2 mm et 4 mm) présentant une largeur de 49 mm et une longueur de 69 mm. Cette éprouvette présente une couleur régulière violette.

Les coordonnées chromatiques de cette éprouvette, mesurées sur la partie épaisse de cette dernière (4 mm), sont alors les suivantes :

$L^* = 29,6$
$a^* = 7,3$
$b^* = -16,2$

EXEMPLE 10

Cet exemple illustre l'utilisation des produits de l'invention en cosmétique pour la préparation d'un crayon de maquillage. Les éléments constituants sont utilisés dans les proportions suivantes :

| Constituants | % en poids |
|---|---|
| A | |
| SILBIONE HUILE 70633 V30 | 40 |
| B | |
| Cire d'abeille | 15 |
| Cire de carnauba | 7 |
| Ozocérite | 7 |
| Paraffine | 20 |
| Huile de vaseline | q.s.p. 100 |
| Alcool cétylique | 1 |
| C | |
| Silicate $BaCuSi_2O_6$ (exemple 3) | q.s. |
| Oxyde de titane | q.s. |

Les éléments du mélange B sont fondus et homogénéisés à 80±2°C, puis maintenus au bain thermostaté régulé à 60±2°C. Le silicate et l'oxyde de titane sont dispersés dans l'huile SILBIONE; ce mélange est placé au bain thermostaté à 60±2°C. Le mélange B est ensuite ajouté. Après homogénéisation, l'ensemble est coulé dans un moule siliconé.

**Revendications**

1. Silicate à base d'alcalino-terreux et de cuivre du type $MCuSi_4O_{10}$ ou $MCuSi_2O_6$, M désignant l'alcalino-terreux, M et Cu pouvant être substitués, caractérisé en ce qu'il se présente sous forme d'agglomérats désagglomérables de taille moyenne d'au plus 20μm, constitués soit de grains monocristallins ou majoritairement monocristallins, soit d'agrégats eux-mêmes constitués de particules monocristallines.

2. Silicate à base d'alcalino-terreux, de titane et de cuivre du type $MCuT:Si_3O_9$ M désignant l'alcalino-terreux, M et Cu pouvant être substitués, caractérisé en ce qu'il se présente sous forme d'agglomérats désagglomérables de taille moyenne d'au plus 20μm, constitués soit de grains monocristallins ou majoritairement monocristallins soit d'agrégats eux-mêmes constitués de particules monocristallines.

3. Silicate selon la revendication 1 ou 2, caractérisé en ce que les grains ou les aggrégats ont une taille moyenne comprise entre 1 et 3μm.

4. Silicate selon l'une des revendications précédentes, caractérisé en ce que sa granulométrie, après désagglomération, est d'au plus 5μm, plus particulièrement d'au plus 3μm.

5. Silicate selon l'une des revendications précédentes, caractérisé en ce que l'alcalino-terreux est le baryum.

6. Silicate selon l'une des revendications précédentes, caractérisé en ce que l'alcalino-terreux est substitué par une terre rare.

7. Silicate selon l'une des revendications précédentes, caractérisé en ce que le cuivre est substitué par un alcalin ou par le zinc, le nickel, le cobalt ou le manganèse.

8. Silicate selon l'une des revendications 1 à 5, caractérisé en ce qu'il répond à la formule $BaCuSi_2O_6$ et en ce qu'il présente les coordonnées chromatiques suivantes : $L^*>30$; $a^*>15$; $b^*<-30$.

9. Silicate selon l'une des revendications 1 à 5, caractérisé en ce qu'il répond à la formule $BaCuSi_4O_{10}$ et en ce qu'il présente les coordonnées chromatiques suivantes : $L>40$; $a<0$; $b<-30$.

10. Pigment coloré notamment bleu ou violet, caractérisé en ce qu'il comprend au moins un silicate selon l'une des revendications précédentes.

11. Procédé de préparation d'un silicate selon l'une des revendications 1 à 10 caractérisé en ce qu'il comporte les étapes suivantes :

   - on mélange un sol de silice ou un silicate et éventuellement un sol de titane avec des sels des autres éléments constitutifs du silicate à l'exception de l'oxygène;
   - on sèche le mélange ainsi obtenu;
   - on calcine le produit obtenu.

12. Procédé selon la revendication 11, caractérisé en ce qu'on sèche le mélange par atomisation.

13. Procédé selon la revendication 11 ou 12, caractérisé en ce qu'on désagglomére le produit calciné.

14. Procédé selon l'une des revendications 11 à 13, caractérisé en ce qu'on utilise des nitrates comme sels.

15. Utilisation des silicates ou des pigments colorés selon l'une quelconque des revendications 1 à 9 dans des matières plastiques, des peintures, des lasures, des caoutchoucs, des céramiques, des glaçures, des papiers, des encres, des produits cosmétiques, des teintures et des revêtements stratifiés.

16. Compositions de matière colorées notamment du type plastiques, peintures, lasures, caoutchoucs, céramiques, glaçures, papiers, encres, produits cosmétiques, teintures et revêtements stratifiés, caractérisées en ce qu'elles comprennent des silicates ou des pigments colorés tels que définis à l'une quelconque des revendications 1 à 9.

**Patentansprüche**

1. Silicat auf der Basis von Erdalkalimetallen und Kupfer vom Typ $MCuSi_4O_{10}$ oder $MCuSi_2O_6$, worin M das Erdalkalimetall bezeichnet und M und Cu substituiert sein können, dadurch gekennzeichnet, daß es in Form von deagglomerierbaren Agglomeraten der mittleren Größe von höchstens 20 µm vorliegt und entweder aus monokristallinen oder zum größten Teil monokristallinen Körnern oder aus den Aggregaten selbst besteht, die ihrerseits aus monokristallinen Teilchen gebildet werden.

2. Silicat auf der Basis von Erdalkalimetallen, Titan und Kupfer vom Typ $MCuTiSi_3O_9$, worin M das Erdalkalimetall bezeichnet und M und Cu substituiert sein können, dadurch gekennzeichnet, daß es in Form von deagglomerierbaren Agglomeraten der mittleren Größe von höchstens 20 µm vorliegt und entweder aus monokristallinen oder zum größten Teil monokristallinen Körnern oder aus den Aggregaten selbst besteht, die ihrerseits aus monokristallinen Teilchen gebildet werden.

3. Silicat nach Anspruch 1 oder 2, dadurch gekennzeichnet, daß die Körner oder die Aggregate eine mittlere Größe zwischen 1 µm und 3 µm besitzen.

4. Silicat nach einem der vorstehenden Ansprüche, dadurch gekennzeichnet, daß seine Granulometrie nach dem Deagglomerieren höchstens 5 µm, vorzugsweise höchstens 3 µm beträgt.

**5.** Silicat nach einem der vorstehenden Ansprüche, dadurch gekennzeichnet, daß das Erdalkalimetall Barium ist.

**6.** Silicat nach einem der vorstehenden Ansprüche, dadurch gekennzeichnet, daß das Erdalkalimetall durch eine seltene Erde substituiert ist.

**7.** Silicat nach einem der vorstehenden Ansprüche, dadurch gekennzeichnet, daß das Kupfer durch ein Alkalimetall oder durch Zink, Nickel, Cobalt oder Mangan substituiert ist.

**8.** Silicat nach einem der Ansprüche 1 bis 5, dadurch gekennzeichnet, daß es der Formel $BaCuSi_2O_6$ entspricht und daß es die folgenden chromatischen Koordinaten aufweist: L*>30;a*>15;b*<-30.

**9.** Silicat nach einem der Ansprüche 1 bis 5, dadurch gekennzeichnet, daß es der Formel $BaCuSi_4O_{10}$ entspricht und daß es die folgenden chromatischen Koordinaten aufweist: L>40;a<0;b<-30.

**10.** Farbpigment, insbesondere von blauer oder violetter Farbe, dadurch gekennzeichnet, daß es mindestens ein Silicat nach einem der vorstehenden Ansprüche umfaßt.

**11.** Verfahren zur Herstellung eines Silicates nach einem der Ansprüche 1 bis 10, dadurch gekennzeichnet, daß es die folgenden Stufen umfaßt:

- man mischt ein Kieselerde-Sol oder ein Silicat und gegebenenfalls ein Sol von Titan mit den Salzen der anderen wesentlichen Elemente des Silicates, mit Ausnahme von Sauerstoff,
- man trocknet die auf diese weise erhaltene Mischung,
- man kalziniert das erhaltene Produkt.

**12.** Verfahren nach Anspruch 11, dadurch gekennzeichnet, daß man die Mischung durch Versprühen trocknet.

**13.** Verfahren nach Anspruch 11 oder 12, dadurch gekennzeichnet, daß man das kalzinierte Produkt deagglomeriert.

**14.** Verfahren nach einem der Ansprüche 11 bis 13, dadurch gekennzeichnet, daß man als Salze Nitrate verwendet.

**15.** verwendung der Silicate oder der Farbpigmente nach irgendeinem der Ansprüche 1 bis 9 in Kunststoffen, Anstrichstoffen, Lasuren, Gummiartikeln, Keramiken, Glasuren, Papieren, Tinten, kosmetischen Produkten, Färbemitteln und Schichtüberzügen.

**16.** Farbstoffzusammensetzungen, insbesondere vom Typ der Kunststoffe, Anstrichstoffe, Lasuren, Gummiartikel, Keramiken, Glasuren, Papiere, Tinten, kosmetischen Produkte, Färbemittel und Schichtüberzüge, dadurch gekennzeichnet, daß sie Silicate oder Farbpigmente wie in irgendeinem der Ansprüche 1 bis 9 definiert umfassen.


**Claims**

**1.** A silicate, based on an alkaline-earth and copper, of type $MCuSi_4O_{10}$ or $MCuSi_2O_6$, M representing the alkaline-earth, M and Cu possibly being substituted, characterized in that it is in the form of deagglomeratable agglomerates with an average size of at most 20 μm, constituted either by mono-crystalline or mainly mono-crystalline grains or by aggregates which are themselves constituted by mono-crystalline particles.

**2.** A silicate based on an alkaline-earth, titanium and copper, of type $MCuTiSi_3O_9$, M representing the alkaline-earth, M and Cu possibly being substituted, characterized in that it is in the form of deagglomeratable agglomerates with an average size of at most 20 μm, constituted either by mono-crystalline or mainly mono-crystalline grains or by aggregates which are themselves constituted by mono-crystalline particles.

**3.** A silicate according to claim 1 or claim 2, characterized in that the grains or aggregates have an average size which is in the range 1 μm to 3 μm.

**4.** A silicate according to any one of the preceding claims, characterized in that its grain size, after deagglomeration, is at most 5 μm, more particularly at most 3 μm.

5. A silicate according to any one of the preceding claims, characterized in that the alkaline-earth is barium.

6. A silicate according to any one of the preceding claims, characterized in that the alkaline-earth is substituted by a rare earth.

7. A silicate according to any one of the preceding claims, characterized in that the copper is substituted by an alkali or by zinc, nickel, cobalt or manganese.

8. A silicate according to any one of claims I to 5, characterized in that it has formula $BaCuSi_2O_6$ and in that it has the following chromaticity co-ordinates:

$$L^*>30; a^*> 15; b^*<-30.$$

9. A silicate according to any one of claims 1 to 5, characterized in that it has formula $BaCuSi_4O_{10}$ and in that it has the following chromaticity co-ordinates:

$$L>40; a<0; b<-30.$$

10. A blue or violet pigment, characterized in that it comprises at least one silicate according to any one of the preceding claims.

11. A process for the preparation of a silicate according to any one of claims 1 to 10, characterized in that it comprises the following steps:

- mixing a silica sol or a silicate and optionally a titanium sol with salts of the other constituent elements of the silicate, with the exception of oxygen;
- drying the mixture obtained;
- calcining the product obtained.

12. A process according to claim 11, characterized in that the mixture is dried by spray drying.

13. A process according to claim 11 or claim 12, characterized in that the calcined product is deagglomerated.

14. A process according to any one of claims 11 to 13, characterized in that the salts used are nitrates.

15. Use of coloured silicates or pigments according to any one of claims 1 to 9 in plastics materials, paints, finishes, rubbers, ceramics, glazes, papers, inks, cosmetics, dyes or laminated coatings.

16. Coloured compositions, in particular plastics, paints, finishes, rubbers, ceramics, glazes, papers, inks, cosmetics, dyes or laminated coatings, characterized in that they comprise coloured silicates or pigments as defined in any one of claims 1 to 9.